Europäisches Patentamt

European Patent Office

Office européen des brevets

(19)

(11) EP 0 878 191 A2

(12) **EUROPÄISCHE PATENTANMELDUNG**

(43) Veröffentlichungstag:
18.11.1998 Patentblatt 1998/47

(51) Int. Cl.$^6$: **A61K 31/135**, A61K 47/14,
A61K 47/38

(21) Anmeldenummer: 98107131.9

(22) Anmeldetag: 20.04.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 22.04.1997 DE 19716905

(71) Anmelder:
IIP Institut für Industrielle Pharmazie
Forschungs- und Entwicklungsgesellschaft
mbH
55442 Stromberg (DE)

(72) Erfinder:
• Ludwig, Gerhard, Dr.
55442 Stromberg (DE)
• Röhrich, Tillmann, Dr.
79104 Freiburg (DE)

(74) Vertreter:
Nöth, Heinz, Dipl.-Phys.
Patentanwalt,
Mozartstrasse 17
80336 München (DE)

(54) **Stabile wässrige Lösung auf der Grundlage von Selegilin und Vorrichtung zu ihrer verabreichung**

(57) Eine sirupähnliche Lösung, enthaltend das L-(-)-Isomere des Selegilins sowie weitere Hilfsstoffe, wie Konservierungsmittel, Stabilisatoren, Aromastoffe, Süßstoffe und den Rest gereinigtes Wasser, sowie seine Verwendung als Anti-Parkinson-Mittel und eine Vorrichtung zur Verabreichung der Lösung über eine Magensonde.

EP 0 878 191 A2

Printed by Xerox (UK) Business Services
2.16.6/3.4

**Beschreibung**

Die Erfindung betrifft eine stabile wäßrige Lösung nach dem Oberbegriff des Patentanspruches 1 sowie eine Vorrichtung zu ihrer Verabreichung nach dem Oberbegriff des Patentanspruches 6, wie aus der DE 44 28 444 A1 bekannt. Die bekannte wäßrige Lösung auf der Grundlage von L-Selegilin soll u.a. auch zur Behandlung von Alzheimer-Erkrankung zum Einsatz kommen. Eine derartige Lösung wurde jedoch bis jetzt noch nicht in der Praxis verwendet, weil es bislang nicht gelungen ist, eine Lösung mit ausreichender Stabilität (Lagerfähigkeit) herzustellen.

Die UPAC-Bezeichnung der L-(-)-Form lautet:

R-(-)-N-Methyl-N-(1-phenyl-2-propyl)-2-propinylamin

Es ist bekannt, Selegilin als Wirkstoff enthaltende Tabletten zur Behandlung der Parkinson'schen Krankheit (Paralysis agitans) zu verwenden, wobei Selegilinhydrochlorid-Tabletten mit jeweils 5 mg oder 10 mg des Wirkstoffs zum Einsatz kommen. Schwierigkeiten bereitet hierbei die Dosierung des Wirkstoffs durch Zerteilen der Tablette. Ferner ist die Stabilität der bekannten Tabletten begrenzt. Die auf dem Markt befindlichen Selegilinhydrochlorid-Tabletten können im Regelfall bei Raumtemperatur bis 36 Monate gelagert werden, ohne daß die Zersetzung des Selegilins zu Methamphetamin die 0,5 % - Grenze überschreitet. Bei Lagerbedingungen in Klimazone 2 (Temperatur 25° C, und Feuchtekontrolle bei 60 % relative Feuchte) wurde die spezifizierte Grenze für Methamphetamin von 0,5 % nach 20 Monaten Lagerung erreicht. Bei Lagerung unter Streßbedingung (Temperatur 40° C und Feuchtekontrolle mit 75 % relativer Feuchte) ergab sich ein Überschreiten der spezifizierten Grenze von 0,5 % bereits nach drei Monaten.

Die durch die geschilderte Erfindung gelöste Aufgabe besteht darin, eine Darreichungsform zu schaffen, mit welcher eine genaue Dosierung des Wirkstoffes und eine erhöhte Stabilität (Lagerfähigkeit) des Präparats erreicht wird.

Im Hinblick auf die Stabilität der Lösung und der Wirksamkeit der Konservierungsmittel ist der pH-Wert der Lösung auf ca. 3,5 bis 5,0 eingestellt. Die Lösung besitzt eine sirupähnliche Konsistenz mit einer Viskosität von etwa 10 - 20 mPas. Der Anteil der Konservierungsmittel, welche Benzoate sind, beträgt 0,95 bis 1,05 mg/ml. Insbesondere kommen als Konservierungsstoffe Methylparahydroxybenzoat und Propylparahydroxybenzoat zum Einsatz. Der Anteil an Methylparahydroxybenzoat beträgt bevorzugt 0,76 mg/ml bis 0,84 mg/ml und insbesondere 0,8 mg/ml. Der Anteil an Propylparahydroxybenzoat beträgt bevorzugt 0,19 mg/ml bis 0,21 mg/ml und insbesondere 0,2 mg/ml.

Die Lösung weist eine Dichte von 1,001 bis 1,003 mg/ml auf. Die Lösung enthält neben den Konservierungsstoffen noch einen Stabilitätshilfsstoff bzw. Stabilitätshilfsstoffe in der Größenordnung von 12,0 mg/ml. Ein bevorzugter Stabilitätshilfsstoff ist Methylhydroxypropylcellulose. Beispielsweise kann Methylhydroxypropylcellulose zum Einsatz kommen, deren durchschnittlicher Substitutionsgrad der Methylgruppen 28 - 30 % und deren durchschnittlicher Substitutionsgrad der Hydroxypropylgruppen 7 - 12 % betragen. Die Viskosität bei einer 2-%igen Konzentration in Wasser beträgt bevorzugt 50 mPas. Ein auf dem Markt befindlicher Stabilitätshilfsstoff ist beispielsweise Methocel E 50 (Warenzeichen).

Der Anteil des Wirkstoffes, nämlich des L-(-)-Isomeres des Selegilins, insbesondere Selegilinhydroxids, beträgt 0,90 - 1,10 mg/ml und insbesondere 1,0 mg/ml.

Ferner können Süßstoffe und Aromastoffe in einer Größenordnung von 2mg/ml in der Lösung enthalten sein. Als Süßstoffe eignen sich Cyclamate, insbesondere Natriumcyclamat in einem Anteil von 0,1 mg/ml und Saccharin, insbesondere Na-Salz des Saccharins in einem Anteil von 0,9 mg/ml. Aromastoffe, beispielsweise Kirscharoma, Dragoco 9/010426-18053 kann in einem Anteil von 1,0 mg/ml in der Lösung enthalten sein. Der Rest ist gereinigtes Wasser.

Ein bevorzugtes Ausführungsbeispiel hat folgende Zusammensetzung:

| Bestandteil | mg/ml |
|---|---|
| Selegilinhydrochlorid | 1,0 |
| Methylparahydroxybenzoat | 0,8 |
| Propylparahxdroxybenzoat | 0,2 |
| Methylhydroxypropylcellulose | 12,0 |
| Natriumcyclamat | 0,1 |
| Saccharin (Na-Salz) | 0,9 |
| Kirscharoma Dragoco 9/010426-18053 | 1,0 |
| Wasser gereinigt | 986,0 |
| | $\overline{1002,0}$** |

** Dichte 20° C ist 1.002 g/ml

Die Stabilitätsuntersuchungen ergaben folgendes:

Die Lösung (Selegilinhydrochloridsirup 1 mg/ml) kann bei 25° C/60 % relativer Feuchte gelagert werden, ohne daß Methamphetamin oder unbekannte Verunreinigungen detektiert werden können. Aus den Ergebnissen ergibt sich eine Stabilität von mindestens 36 Monaten bei 25° C und 60 % relativer Feuchte. Bei Streßbedingungen (40° C/75 % relativer Feuchte) konnten nach 6 Monaten nur geringe Mengen Methamphetamin und keine weiteren Verunreinigungen festgestellt werden.

Die Stabilitätsdaten für Klimazone 2 und Streßbedingungen sind im folgenden wiedergegeben: Stabilitätsdaten Selegilinhydrochlorid Sirup 1 mg/ml (Reinheitsprüfung)

| Lagerbedingung 25°C/60 % r.F. (Klimazone 2, mit Temperatur und Feuchtekontrolle) | |
|---|---|
| | 12 Monate |
| Methamphetamin | Nicht meßbar |
| unbekannt | Nicht meßbar |
| Summe | ---- |

| Lagerbedingung 40°C/74 % r.F.(Streßbedingungen, mit Temperatur u. Feuchtekontrolle) | | |
|---|---|---|
| | 3 Monate | 6 Monate |
| Methamphetamin | Nicht meßbar | Max. 0,02 % |
| Unbekannt | Nicht meßbar | ---- |
| Summe | ----- | 0,02 % |

Im Vergleich hierzu betragen die Stabilitätswerte einer Tablette mit 5 mg Selegilinhydrochlorid (Reinheitsprüfung):

| Lagerbedingung Raumtemperatur (Klimazone 1, keine Temperatur und Feuchtekontrolle) | | | |
|---|---|---|---|
| | 12 Monate | 24 Monate | 36 Monate |
| Mathamphetamin | Ca. 0.1 % | Ca. 0.3 % | unter 0.5 % |
| Unbekannt | 3 Verunr. unter 0.1% | 3 Verunr. unter 0.1 % | 3 Verunr. unter 0.1 % |
| Summe | Ca. 0.4 % | Ca. 0.6 % | Ca. 0.8 % |

| Lagerbedingung 25°C/60 %r. F. (Klimazone 2, mit Temperatur und Feuchtekontrolle) | | | |
|---|---|---|---|
| | 12 Monate | 24 Monate | 36 Monate |
| Methamphetamin | Ca. 0.2 % | unter 0.5 % | unter 1 % |
| Unbekannt | 1 Verunr. Ca. 0.12 % | 1 Verunr. Ca. 0.15 % | 1 Verunr. Ca. 0.15 % |
| | 2 Verunr. unter 0.1% | 2 Verunr. unter 0.1% | 2 Verunr. unter 0.1% |
| Summe | Ca. 0.5 % | Ca. 0.9 % | Ca. 1.4 % |

| Lagerbedingung 40°C/75 %r. F. (Streßbedingung, mit Temperatur und Feuchtekontrolle) | | | |
|---|---|---|---|
| | 3 Monate | 6 Monate | |
| Methamphetamin | Ca. 0.9 % | Ca. 2 % | |
| Unbekannt | 2 Verunr. unter 0.1% | 2 Verunr. unter 0.1% | |
| Summe | Ca. 1.1 % | Ca. 2.2 % | |

Die erfindungsgemäße Selegilinlösung läßt sich unkompliziert und ohne großen Zeitaufwand herstellen, wobei Standardverfahren der pharmazeutischen Technologie zum Einsatz kommen, wie im folgenden erläutert wird:

In einem heiz- und rührbaren Ansatzbehälter, beispielsweise mit einem Volumen von 2100 Liter wird zunächst gereinigtes Wasser in einer Menge von 300 kg mit 1 kg der Konservierungsstoffe, nämlich 0,8 kg Methylparahydroxybenzoat und 0,2 kg Propylparahydroxybenzoat unter Rühren auf 80 bis 90° C erhitzt, bis eine klare Lösung entsteht. In den noch heißen Ansatz werden 12,0 kg eines stabilitätserhöhenden Hilfsmittels, beispielsweise Methylhydroxypropylcellulose, eingetragen und durch kräftiges Rühren suspendiert. Sodann wird mit 686 kg kaltes und gereinigtes Wasser ergänzt, und die Suspension wird unter Weiterrühren auf 30° C abgekühlt. Dabei entsteht eine klare Lösung.

In diese Lösung werden anschließend der Wirkstoff, nämlich das L-(-)-Isomere des Selegilinhydrochlorids in einer Menge von 1,0 kg, die Süßstoffe Natriumcyclamat in einer Menge von 0,1 kg und Natriumsalz des Saccharins in einer Menge von 0,9 kg sowie der Aromastoff Kirscharoma Dragoco 9/010426-18053 in einer Menge von 1,0 kg eingetragen und unter Rühren gelöst. Anschließend wird der Ansatz mit weiterem gereinigtem Wasser ergänzt und auf das Endgewicht von 1.002,0 kg eingestellt.

In einer geeigneten Filtriervorrichtung, beispielsweise mit einem Polypropylenfiltergewebe mit 20 µm Porenweite, wird die fertige Arzneistofflösung unter Stickstoffüberdruck (1800 bis 2050 h/Pa) in ein Behältnis aus alkalifreiem Glas oder Edelstahl filtriert.

Mit der erfindungsgemäßen Selegilinlösung steht ein oral anwendbares flüssiges (Sirup) Parkinson-Mittel für eine einfache und präzise Dosierung zur Verfügung. Als Dosierhilfe bei der Endabfüllung kann ein Meßbecher mit 0,5 ml-, 1,0 ml-, 2,5 ml- und 5,0 ml - Skalierung entsprechend 0,5 mg, 1,0 mg, 2,5 mg und 5,0 mg Selegilinhydrochlorid verwendet werden. Hierdurch wird eine optimal dosiergenaue Einstellung des Patienten aufgrund der Lösungsdichte von etwa 1 mg/ml ermöglicht.

Die L-(-)-Form des Selegilinhydrochlorids ist therapeutisch wirksam zur Behandlung des Parkinson-Syndroms (Paralysis agitans). Das Parkinson-Syndrom beruht auf einer Degeneration dopaminerger Neurone der Substantia nigra. In der Substantia nigra entsteht aus der Aminosäure Tyrosin der Neurotransmitter Dopamin, der zur Steuerung unbewußter Bewegungsabläufe unerläßlich ist. Durch einen biochemischen Defekt kommt es zum Dopamindefizit in bestimmten Hirnregionen und in der Folge zur Parkinson'schen Krankheit. Im menschlichen Gehirn wird der größte Teil des Dopamin von Monoaminooxidase B(MAO-B) metapolisiert. Die symptomatische Therapie beschränkt sich im allgemeinen darauf, den Dopamin-Mangel durch Gabe von Levodopa (L-Dopa), der Vorstufe des Dopamins, auszugleichen. Nach mehrjähriger Applikation kommt es jedoch zu Fluktuationen und einem Nachlassen der Wirksamkeit von L-Dopa mit einer Verschlechterung des Zustandsbildes. Die L-(-)-Form des Selegilins ist ein irreversibler selektiver Inhibitor, der für den Dopamin-Abbau vorzugsweise verantwortlichen MAO-B in den betroffenen Hirnabschnitten. Der Wirkstoff bewirkt in der Folge einen Anstieg der Dopamin-Konzentration mit einer Verstärkung der bei Morbus Parkinson verminderten dopamingesteuerten Reizweiterleitung. Der Wirkstoff führt zu keiner Veränderung der Sensitivität zentralnervöser Dopamin-Rezeptoren.

Durch die Gabe des MAO-B-Hemmers Selegilin ergibt sich ein erweitertes Therapieprinzip, um den Dopamin-Abbau zu verlangsamen.

Die Zusatzbehandlung mit Selegilin zur Basistherapie mit L-Dopa bewirkt somit eine Potenzierung und Verlängerung der Wirkung von L-Dopa. Selegilin ermöglicht so eine Verminderung der L-Dopa-Dosis bei gleichbleibendem Effekt und kann das gefürchtete Nachlassen der Wirkung von L-Dopa ausgleichen. Dies zeigt sich in einer Verbesserung der Parkinson-Symptomatik und eine Verminderung der Fluktuationen der Symptomatik.

Selegilin ist auch als Monotherapeutikum im Anfangsstadium der Parkinson'schen Krankheit wirksam, wodurch vor allem die Progression der Krankheit verlangsamt wird und ein deutlich späterer Beginn der L-Dopa-Behandlung möglich wird.

Durch die erfindungsgemäße oral verabreichbare Selegilin-Lösung (Sirup) steht eine flüssige Arzneiform für die dosiergenaue Versorgung Parkinson-kranker Patienten zur Verfügung. In bevorzugter Weise erreicht man eine körpergewichtsabhängige Dosierung von 1 mg Wirkstoff pro 10 kg Körpergewicht des Patienten. Ferner ist eine dosiergenaue enterale Versorgung mittels Magensonde möglich. Hierdurch kann einem häufigen Symptom bei Morbus Parkinson, nämlich der Dysphagie (Schluckbeschwerden) Rechnung getragen werden. Die mit Schluckbeschwerden verbundene Aspiration ist die Hauptursache von Morbidität und Mortalität bei dieser Krankheit. Die Pathogenese dieser Schluckbeschwerden ist multifaktoriell. Mit der Magensonde umgeht man die für Patienten mit schweren Schluckbeschwerden problematische bis lebensgefährliche orale Zufuhr von Flüssigkeitnahrung und Medikamenten. Die erfindungsgemäße Selegilin-Lösung garantiert eine einfache zweckmäßige und dosiergenaue Medikation über eine Magensonde. Dabei kann es sich um eine nasogastroduodenale Sonde oder eine endoskopisch perkutan angelegte gastroduodenale Sonde (PEG-Sonde) handeln. Die erfindungsgemäße Selegilin-Lösung läßt sich auf diesem Wege flüssig als Bolusinjektion (Einzelgabe) oder pumpengesteuert zuführen.

Wie oben schon ausgeführt, kann mit der erfindungsgemäßen Selegilin-Lösung die Dosis des verabreichten Wirkstoffes genau an das Körpergewicht des Patienten angepaßt werden. Im Bedarfsfall kann die Dosis äußerst behutsam praktisch stufenlos variiert gegebenenfalls gesteigert werden. Dies ist im Vergleich zu der bisherigen Verabreichungsform der Tablette, welche zerteilt oder in Flüssigkeit suspendiert werden mußte, von erheblichem Vorteil. Mit der Dosierung 1 ml erfindungsgemäßer Selegilin-Lösung läßt sich die gewünschte Arzneigabe von 1 mg Selegilin/10 kg Körpergewicht des Patienten mühelos und genau vorgeben. Insbesondere bei untergewichtigen Patienten ist dies notwendig, da eine zu hohe Selegilin-Gabe die Selektivität des Wirkstoffes als ausschließlichen Hemmer der MAO-B negativ beeinflußt. Die Standarddosierung beträgt für Erwachsene bei Monotherapie 10 ml Lösung entsprechend 10 mg Wirkstoff pro Tag und in Kombination mit einer individuell angepaßten Dosis von L-Dopa 5 ml bis 10 ml Lösung entsprechend 5 bis 10 mg Wirkstoff pro Tag. Mit ansteigender Dosierung kommt es zu einer zunehmenden Hemmung der Monoaminooxidase A (MAO-A). Dies geht mit einer wachsenden Sensitivität gegenüber unerwünschten Herz-Kreislaufwirkungen einher, da der Körper weniger vor dem übermäßigen Anfluten nahrungsbedingter biogener Amine, wie Tyramin, mit seiner blutdrucksteigernden Wirkung geschützt ist. Durch die individuell gewichtsangepaßte Dosierung wird daher eine optimale Verträglichkeit erreicht. Da die erfindungsgemäße Selegilin-Lösung auch oral eingenommen werden kann, werden Einnahmeprobleme, welche mit Tabletten bestanden haben, gelöst. Dies betrifft vor allem die beachtliche Anzahl von Parkinson-Patienten mit leichteren Schluckbeschwerden und die wachsende Zahl von Patienten mit psychisch begründeten Vorbehalten gegen eine Tabletteneinnahme.

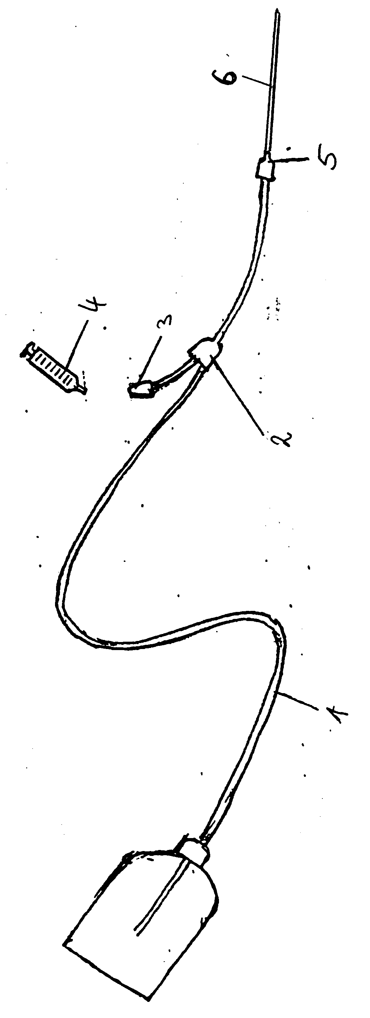

In der Figur ist ein Ausführungsbeispiel zur Erläuterung der Verabreichung der erfindungsgemäßen Selegilin-Lösung mittels Magensonde dargestellt.

Eine Einmalspritze 4, z.B. 5 ml-Spritze mit abgemessenem Dosisvolumen wird über ein Y-Anschlußstück 2 mit paßgenauem Ansatz 3, beispielsweise einem Luer-Verschluß für die Einmalspritze 4 mit einem Magensondenschlauch 1 verbunden. Der paßgenaue Ansatz 3 kann nach dem Entfernen der Einmalspritze 4, beispielsweise mittels eines Plastikkappenschraubverschlusses, dicht verschlossen werden. Durch den Ansatz 3 des Y-Anschlußstückes 2 kann bei Bedarf auch ein Spülmittel zum Spülen des Sondenschlauches zugeführt werden. Am unteren Ende des Y-Anschlußstückes 2 befindet sich ein Verbindungsstück 5, beispielsweise in Form einer Schraubverbindung zur Magensonde 6.

Für die Darreichung wird die Selegilin-Lösung in der gewünschten Dosierung in die Einmalspritze 4 eingegeben und die Einmalspritze auf das Ansatzstück paßgenau aufgesetzt. Die dosierte Selegilin-Lösung kann dann in einfacher Weise dem Magensondenschlauch 1 und damit der Magensonde 6 zugeführt werden. Hierdurch wird eine dosiergenaue enterale Versorgung mittels Magensonde zur Verfügung gestellt.

**Patentansprüche**

1. Stabile wäßrige Lösung auf der Grundlage von L-Selegilin, insbesondere Selegilinhydrochlorid, und pharmazeutisch geeigneten Hilfsstoffen,
   dadurch gekennzeichnet,
   daß sie durch ein Stabilisierungsmittel auf eine Viskosität von 10 bis 20 mPas eingestellt ist und einen pH-Wert von 3,5 bis 5.0 besitzt sowie als Konservierungsmittel 0,95 bis 1,05 mg/ml Benzoate enthält.

2. Lösung nach Anspruch 1, dadurch gekennzeichnet, daß sie 0,76 bis 0,85 mg/ml vorzugsweise 0,8 mg/ml Methylparahydroxybenzoat und 0,19 bis 0,21 mg/ml vorzugweise 0,2 mg/ml Propylparahydroxybenzoat enthält.

3. Lösung nach Anspruch 1 oder 2, dadurch gekennzeichnet, daß sie 0,9 bis 1,1 mg/ml, insbesondere 1,0 mg/ml L-(-)-Selegilinhydrochlorid als Wirkstoff enthält.

4. Lösung nach einem der Ansprüche 1 bis 3, dadurch gekennzeichnet, daß sie zusätzlich L-DOPA enthält, und insbesondere zur Behandlung der Parkinson'schen Krankheit eingesetzt wird.

5. Lösung nach einem der Ansprüche 1 bis 4, dadurch gekennzeichnet, daß sie als Stabilisierungsmittel 12 mg/ml Methylhydroxypropylcellulose aufweist.

6. Vorrichtung zur Verabreichung des Mittels nach einem der Ansprüche 1 bis 5, gekennzeichnet durch eine Magensonde (6), an die ein Magensondenschlauch (1) angeschlossen ist, der ein Anschlußstück (2), insbesondere Y-förmiges Anschlußstück (3), aufweist, welches mit einer Wirkstoff-haltigen Spritze 84) verbunden werden kann.